# EUROPEAN PATENT SPECIFICATION

(11) **EP 0 514 026 B1**
(45) Date of publication and mention of the grant of the patent: **11.03.1998**
(21) Application number: 92303593.5
(22) Date of filing: 22.04.1992
(51) Int. Cl.: A61B 17/12

(54) **Compressive haemostatic belt**
Hämostatischer Druckgürtel
Ceinture hémostatique compressive

(30) Priority: 23.04.1991 JP 122351/91; 06.02.1992 JP 20793/92; 19.02.1992 JP 31236/92; 28.02.1992 JP 9759/92; 26.03.1992 JP 67682/92
(43) Date of publication of application: 19.11.1992
(73) Proprietor: SUMITOMO RUBBER INDUSTRIES LIMITED, Kobe-shi Hyogo-ken (JP)
(72) Inventor: Kawasaki, Atsuko, Akashi-shi, Hyogo-ken (JP); Nakashita, Takefumi, Kobe-shi, Hyogo-ken (JP); Inui, Yoshiharu, Hyogo-ken (JP); Hori, Shinichi, Sakai-shi, Osaka-fu (JP); Sakaki, Toshiaki, Hyogo-ken (JP); Miki, Miyo, Kobe-shi, Hyogo-ken (JP)
(74) Representative: Hillier, Peter

(56) References cited:
- DE-C- 56 336
- DE-C- 571 744
- FR-A- 910 340
- GB-A- 1 206 605
- US-A- 2 104 758
- US-A- 3 171 410
- US-A- 3 467 085
- US-A- 3 633 567
- US-A- 3 670 735
- US-A- 4 275 718

## Description

The present invention relates to a compressive hemostatic belt used to stop bleeding from a catheter insertion wound upon completion of an arterial catheter examination.

Recently, arterial catheter examinations have been made for contrast medium-using diagnosis of hearts or cerebral blood vessels. Cardiac catheter examinations are made by surgical operation in a few cases but in most cases by the so-called catheter puncture method which moves a catheter from the femoral artery or vein in the inguinal region to the heart.

In this examination method, a contrast medium or various medicines are injected through the catheter puncturing the femoral artery or vein in the inguinal region or various preoperative and postoperative examinations are conducted. In this connection, there is a need to compress the catheter insertion wound for a relatively long time in order to stop bleeding from the catheter insertion wound owing to extraction of the catheter from the femoral artery or vein in the inguinal region.

As for such compressive hemostatic method for catheter insertion wounds, it has been common practice for a doctor or nurse to manually compress a catheter insertion wound for about 15 minutes, apply gauze to the catheter insertion wound, apply 3 or 4, 70-cm long 5-cm wide fabric adhesive plaster over said gauze to compress said catheter insertion wound from above said gauze, placing a sand bag having a controlled weight of 500 - 1000 kg, and fix said sand bag against moving by said fabric adhesive plaster, such fixed state being maintained for 12 - 24 hours.

However, in the conventional method described above, the use of adhesive plaster as means for fixing the gauze applied to the catheter insertion wound and also fixing the sand bag placed thereon after extraction of the catheter causes such drawbacks to the patient as a stiffening feel, pain and itch and favours the development of dermatitis and vesicular exanthema.

The sand bag tends to slip off when the patient lying on his back tilts his body even slightly, and such deviation of the sand bag results in the sand bag deviating from the compressed area, making the hemostatic effect imperfect, leading to other drawbacks, such as ecchymoma.

On the other hand, in order to solve said problems, Japanese Patent Application Disclosure No. 92746/1985 or Japanese Patent Application Disclosure No. 198139/1985 suggests a compressive hemostatic belt formed of stretch fabric as means for compressive hemostatic means for catheter insertion wounds to replace the adhesive plaster and sand bag.

Such compressive hemostatic belt, however, is constructed to make it difficult for a user to wrap it and to visually ascertain the level of its compressive force on the catheter insertion wound and makes it necessary for him to rewrap it when adjusting the compressive force; thus, the loading state of the belt is unstable.

Further, since the belt uses an expensive stretch textile fabric, its production cost is very high, making it difficult to throw it after use when there is a hygienic problem of causing infectious diseases due to adhesion of blood. Therefore, to prevent such hemoinfectious diseases, after each use of such compressive hemostatic belt, it has to be washed and sterilized, imposing limitations not only from a hygienic standpoint but also from the standpoint of enhancing labour saving for nurses.

A hemostatic belt is disclosed in GB-A-120605 comprising a flexible support for a sterile dressing package and a bladder of stretchable rubber positioned on the opposite face of the support for expansion in a pocket formed by the support material and a wall of flexible material.

The present invention provides a compressive hemostatic belt comprising a strip of non-stretch or low-stretch fabric, a bag adapted to be expanded by being filled with fluid, said bag being attached to said strip at a predetermined position by means of a pocket formed on said strip and capable of holding said bag, characterised in that an opening is formed in the surface of said pocket opposed to a region where bleeding is to be stopped.

The bag attached to the strip at the predetermined position has a pump and a pressure gage connected thereto through a check valve, and operator operates the pump while watching the pressure gage to fill the bag with fluid to expand said bag so as to compress an area where bleeding is to be stopped. Since a nonstretch or low-stretch textile fabric is used for the strip, the development of a stiffening feel, pain or itch can be avoided and dermatitis or bubbles are prevented. The use of the bag which is expanded by being filled with fluid as compressing means provides a narrower range of compression, ensuring a hemostatic function for compressing only a catheter insertion wound while causing almost no compressive feeling to be transmitted to the other areas.

Further, since the pressurizing mechanism is constructed by connecting, through a check valve, a pump and a pressure gage to the bag which is expansible by being filled with fluid, it is possible to adjust the compressive force to be applied to a catheter insertion wound and to maintain such adjusted force for a long time by the check valve. Further, since compressing and fixing are simultaneously effected by the bag which is expansible by being fixed with fluid, there is provided a compressive hemostatic belt whose operability is satisfactory and which does not require a sand bag or the like and is compact and easy to carry about. The components other than the pump and pressure gage are much cheaper than conventional parts, allowing the belt to be thrown away once it has been used; thus, noteworthy effects from the standpoint of improving health control and promoting labor saving are attained.

A rigid case for preventing the bag from expanding to the side opposite to the area where bleeding it to be stopped may be installed, if necessary, between the bag and the strip, said rigid case having an inner flange around its open end to surround said bag or balloon. The inner flange of said rigid case may be a separate body with the intended compression area cut out in doughnut form and may be attached to the open end of said rigid case.

With the inner flange formed around the open end of the rigid case to surround the balloon, the balloon can be reliably expanded to the area where bleeding is to be stopped with the inner flange of the rigid case prevents the balloon from deviating outside. Therefore, there is no possibility of the bag from deviating from the predetermined position and said area can be positively compressed by the bag to provide a perfect hemostatic effect. Further, if the inner flange of the rigid case is a separate body with the compressive area cut out to suit the affected area where bleeding is to be stopped and is attached to the open end of said rigid case, doughnut-shaped inner flanges with various compressive areas cut out may be prepared, making it possible to adjust the compressive area and to prevent stagnation otherwise caused by wide area compression.

Since such compressive hemostatic belt is applied to a patient who is lying on his side upon completion of catheter examination, the wrapping operation of the strip is difficult to perform depending upon the location where the belt is applied. Further, the strip tends to develop wrinkles and kinks in its portion applied to the patient's waist and hip. Such wrinkles and kinks give the patient an unpleasant feeling and the compression of the wound is made insufficient by the wrinkles being smoothed after the wrapping of the strip. If the strip is wrapped with a greater force in an effort to smooth the wrinkles, the wound is compressed more than necessary.

In an embodiment of the invention a reinforcing sheets is attached to the portion of the strip which covers the area of the body extending from the waist to the hip when the belt is applied. This embodiment provides a compressive hemostatic belt which does not develop wrinkles when applied and which is capable of exerting a stabilised compressive force.

The reinforcing sheet is preferably 10 - 90 cm long and is placed laterally of the pocket of the strip.

The provision of the reinforcing sheet on the portion of the strip which covers the area of the patient's body extending from the waist to the hip prevents wrinkles or kinks from being developed in this portion when the strip is wrapped, and the strip can be reliably wrapped without having to exert a very great force; thus, a degree of tightening which is suited to the patient can be obtained.

By suitably changing the place of application of the reinforcing sheet and the reinforcing area, various degrees of tightening suited to persons of various body types, such as children, slim persons and fat persons can be obtained.

Thereby, the wound can be reliably compressed and there is no possibility of giving the patient an unpleasant feeling due to kinks or the like. Further, since the force required for wrapping is not so great, the labor of nurses or the like for installing the belt can be reduced and stagnation and ecchymoma due to greater compression than necessary can be avoided.

The invention will now be described in more detail and by way of example only with reference to the drawings in which:
Fig.1 is a front view showing a compressive hemostatic belt according to an embodiment of the present invention;
Fig.2 is an explanatory view showing an example of the way said compressive hemostatic belt is wrapped;
Fig.3 is a longitudinal sectional view showing the state existing before balloon expansion, taken at the pocket position on a compressive hemostatic belt according to the invention having a passage formed in a pocket fabric;
Fig.4 is a longitudinal sectional view showing the state existing during balloon expansion, taken at the pocket position on the compressive hemostatic belt of Fig.3;
Fig.5 is an explanatory view showing an example of the way said compressive hemostatic belt is wrapped;
Fig.6 is a front view of a compressive hemostatic belt according to another embodiment of the invention;
Fig. 7 is a longitudinal sectional view showing the compressive portion of said compressive hemostatic belt;
Fig.8 is an explanatory view showing how the compressive hemostatic belt is used;
Fig.9 is a longitudinal sectional view showing the compressive portion of said compressive hemostatic belt during use;
Fig.10 is a longitudinal sectional view of a rigid case used for said compressive hemostatic belt;
Fig.11 is a longitudinal sectional view of another example of the rigid case used for said compressive hemostatic belt;
Fig.12 (a) is a plan view of the compressive hemostatic belt of the present invention;
Fig.12 (b) , (c), and (d) are sectional views taken along the line A - A in Fig. 21 (a);
Fig.13 (a) is a front view of a human body showing how the compressive hemostatic belt of the invention is used; and
Fig.13 (b) is a rear view of a human body showing how the compressive hemostatic belt of the invention is used.

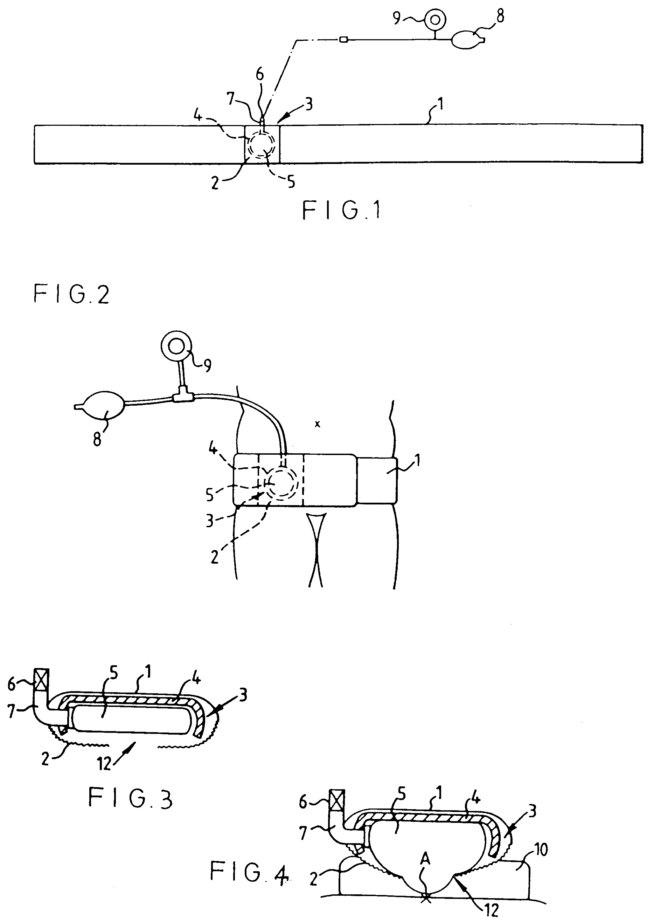

Figs.1 and 3 show a compressive hemostatic belt according to an embodiment of the invention. The numeral 1 denotes a strip made of a nonstretch or low-stretch textile fabric formed with a pocket 3 by sewing a pocket fabric 2 in the form of a stretch textile fabric there to at a predetermined position. The numeral 4 denotes a rigid case which is a bowl-shaped rigid member made of synthetic resin or the like, said rigid case being received in the pocket 3 of the strip 1 with its open side directed to the pocket fabric 2. The numeral 5 denotes a bag which can be expanded by being filled with a suitable fluid (a gas, such as air or nitrogen gas or a liquid including a gel) , for example, a balloon made of rubber, received in the pocket 3 of the strip 1 through the rigid case 4, with a fluid feed tube 7 projecting outward and having a check valve 6. The fluid tube 7 of the balloon 5 has a manually operable pump 8 and a pressure gage 9 connected thereto. Thus, by operating the pump 8 while watching the pressure gage 9, fluid is filled into the balloon 5 through the fluid feed tube 7 to expand said balloon 5.

The way of using the compressive hemostatic belt of the present invention will now be described.

As shown in Fig.4, gauze 10 is placed on a catheter insertion wound A after extraction of the catheter, and then the belt is placed thereon such that the pocket fabric 2 of the pocket 3 receiving the balloon 5 is directed to the gauze 10. Then, for example, as shown in Fig.2, the strip 1 is wrapped around the waist of the patient more than one turn, the end being fixed in place by utilising fixing means, such as adhesive plaster.

Upon completion of the wrapping of the strip 1, the manually operable pump 8 and pressure gage 9 are connected to the fluid feed tube 7 of the balloon 5 and the pump 8 is operated while watching the pressure gage 9, so as to fill the balloon 5 with the fluid, thereby expanding the balloon 5 toward the catheter insertion wound A with the rigid case 4 preventing the balloon 5 from expanding toward the side opposite to the catheter insertion wound A, whereby the catheter insertion wound A is compressed through the gauze 10 and at the same time the strip 1 is firmly secured to the patient.

At this time, by adjusting the compressive force by changing the amount of liquid or gas injected into the balloon 5 in accordance with the bodily shape of the patient by the pump 8 while watching the pressure gage 9, a compressively hemostatic effect suited for the patient can be obtained, and the thus adjusted compressive force can be maintained for a long time by the check valve 6. it is also possible to employ automatic compressive force control using a computer, as the need arises.

In addition, in the above embodiment, the strip 1 is described as formed of a nonstretch textile fabric, but it may be formed of a low-stretch textile fabric, and the pocket 3 is described as formed by sewing a fabric 2 which is a stretch textile fabric, it may be formed by fabricating the strip 1 in bag form to provide a pocket 3 at a predetermined position to receive the rigid case 4 and balloon 5 therein. Even in the case where the strip 1 is fabricated in bag form using a nonstretch or low-stretch textile fabric, as described above, the compressive force exerted by the expansion of the balloon 5 on the catheter insertion wound A is sufficient. If, however, the surface of the pocket 3 of the strip 1 receiving the balloon 5 opposite to the catheter insertion wound A made of stretch fiber, the compressive force on the catheter insertion wound A can be increased.

In the above embodiment, the balloon 5 is received in the pocket 3 of the strip 1 through the rigid case 4 for preventing the expansion of the balloon 5 toward the side opposite to the catheter insertion wound A. However, the balloon 5 may be received naked in the pocket 3 of the strip 1. In this case also, the balloon 5 can be expanded toward the catheter insertion wound A while the strip 1 prevents it from expanding toward the side opposite to the catheter insertion wound A.

When the balloon 5 is expanded with liquid, as compared with the case of expanding it with gas, the liquid less tends to leak through the balloons 5 and the check valve 6 attached thereto, and even if it leaks, the leakage can be more easily detected and the compressive force can be maintained at a constant value.

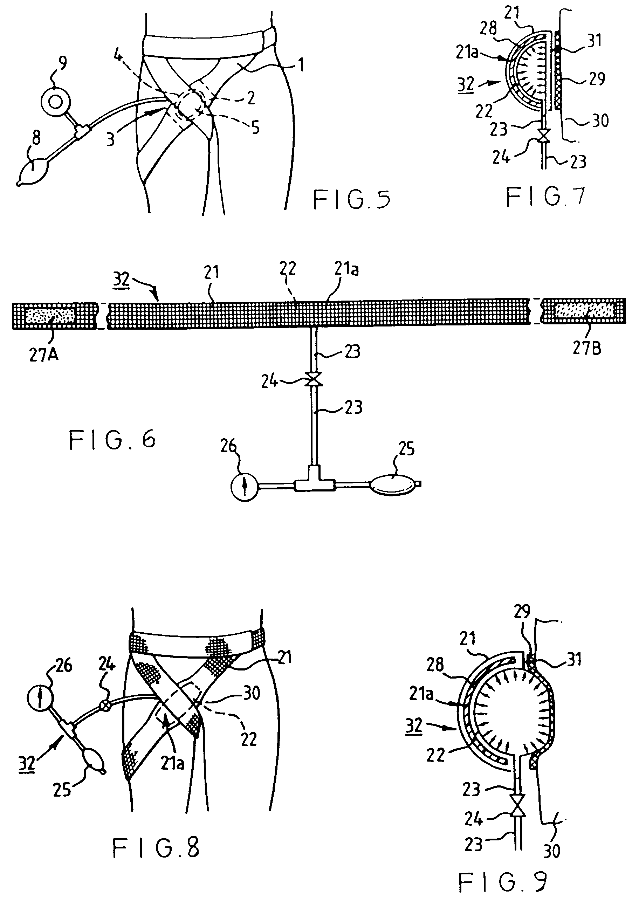

In the above embodiment, as shown in Fig.2 the strip 1 is wound at right angles with the body axis; however, the invention is not limited there to. Besides the wrapping method shown in Fig. 2, the strip 1 may be wrapped X-wise as shown in Fig.5.

According to said compressive hemostatic belt, since the catheter insertion wound A can be compressed by utilizing the expansion of the balloon 5 as described above, the catheter insertion wound A alone can be compressed, with almost no compressed feeling transmitted to the other areas.

Further, since a nonstretch or low-stretch fabric is used for the strip 1, the occurrence of a stiff feeling or itch is avoided and dermatitis and bubbles are prevented.

The above relates to an embodiment of the invention but the invention is not limited thereto and various changes in engineering design are possible within the scope of the invention.

First, while the preceding embodiment uses rubber as a material for the bag, rubber may be replaced by hardly fluid-permeable materials which are soft at ordinary temperature, including thermoplastic materials, such as polyethylene terephthalate, soft vinyl chloride, vinylidene chloride, nylon 6, nylon 6-6 and nylon 12, and composite materials such as polyethylene terephthalate and polyethylene.

Further, as shown in Fig.3, the pocket 3 is formed with an opening 12 to expose the balloon 12. If the pocket 3 is formed with such opening 12, as shown in Fig.4 it is possible to allow the balloon 5 to expand such that it partly projects through the opening 12, thereby increasing the compressive force on the catheter insertion wound A.

In the above embodiment, the balloon 5 or bag 12 is received in the pocket 3 of the strip 1 to thereby attach the balloon 5 or bag 11 to the strip 1; however, the balloon 5 or bag 11 may be attached to the strip 1 at a predetermined position by fastening means such as an adhesive agent or fastener.

Another embodiment of the invention will now be described with reference to Figs.6 through 9.

A compressive hemostatic belt 31 according to the invention comprises a bag-like strip 21 formed of a knit fabric, woven fabric or nonwoven fabric of nonstretch fiber, such as cotton or polyester or nonstretch or lowstretch fiber, such as polyamide, a rubber balloon 22 inserted in a pocket 21a formed at a predetermined position in said strip 21 through a bowl 28 of synthetic resin, said two members forming compressive means for an area where bleeding is to be stopped, e.g., a catheter insertion wound. The strip 21 may be formed in its entirety of a knit fabric or woven fabric of nonstretch or low-stretch fiber or may have its portion opposed to the catheter insertion wound 30 made of stretch fiber, e.g., LIKURA (phonetically) (elastic fiber produced by Du pont de Nemours & Co.). If the portion opposed to the catheter insertion wound 30 is made of stretch fiber as described above, the rubber balloon 22 can be expanded more easily than when the whole of the strip 21 is made of nonstretch fiber, and the compressive force on the insertion wound 30 is further increased. The rubber balloon 22 may be received in the pocket 21a of the strip 21 with the check valve 24 exposed, and in order to prevent the rubber balloon 22 from expanding toward the side opposite to the insertion wound 30, as shown in Fig.7 it may be received in the pocket 21a of the strip 21 through a bowl 28 made of synthetic resin. A manually operable air pump 25 and a pressure gage 26 are connected through said check valve 24 to the air feed tube 23 leading to the rubber balloon 22. By operating the air pump 25 while watching the pressure gage 26, the pressure in the rubber balloon 22 is maintained at a value suitable for compressing the insertion wound 30 for a predetermined period of time. An automatic pressure control system using a computer may be employed as the need arises.

An embodiment wherein upon completion of heart catheter examination, the compressive hemostatic belt according to the invention is used to stop bleeding from the insertion wound 30 in the inguinal region, will now be described with reference to Figs. 8 and 9.

Gauze 29 is placed on the insertion wound 30 subsequently to extraction of the catheter and the compressive hemostatic belt is wrapped thereon. That is, the pocket 21a of the strip 21 having the balloon 22 received therein is placed on the insertion wound 30, and strip 21 is passed from the inguinal region of the lower limb over the outer side, back side and inner side of the thigh and from the crotch over the front side of the thigh and crossed X-wise in the inguinal region, the remaining portion being wrapped in one or more layers around the trunk, the remaining portion of the strip 21 of the strip 21 being fixed in position by fixing means such as directionless fabric fasteners 27A and 27B. Thereafter, the air pump 25 is operated while watching the pressure gage 26 to expand the rubber balloon 22, as shown in Fig.8, to produce a compressive force to position and fix the compressive portion 31 on the insertion wound 30. The above description refers to an example in which the present inventive device is used to stop bleeding from the insertion wound 30 in the inguinal region of the lower limb; however, by controlling the abutting position of the rubber balloon 22 and the wrapping position of the compressive hemostatic belt 32, the compressive hemostatic effect can be exerted in any portion of the body other than the inguinal region of the lower limb.

Another embodiment of the invention will now be described with reference to the drawings.

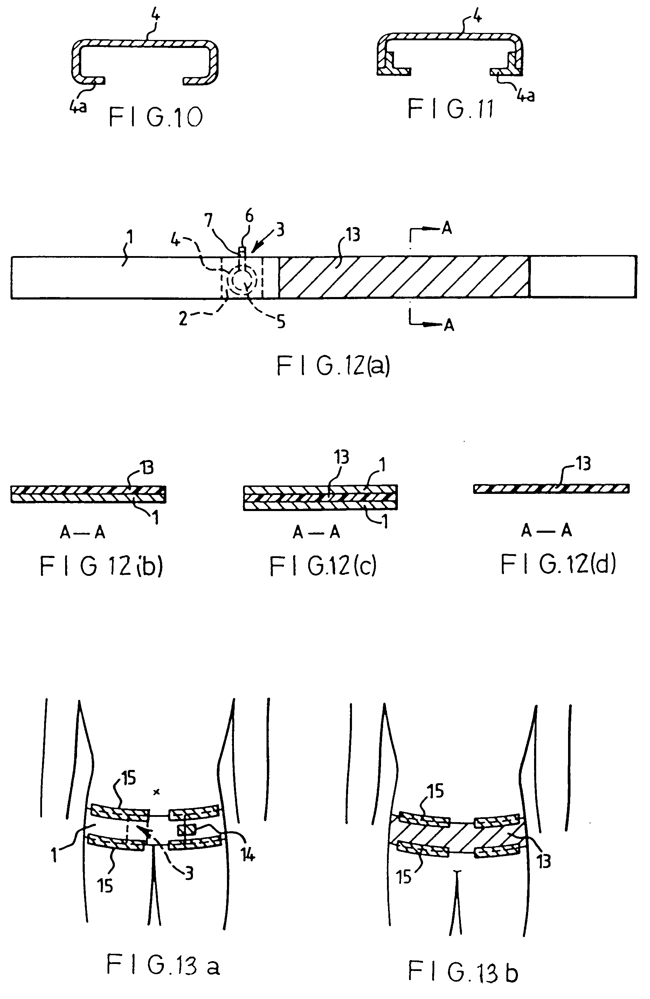

Figs. 10 and 11 show another embodiment of the invention which is the same as the compressive hemostatic belt of Figs. 1 through 4 except the following, and like reference characters are applied to like parts to omit a repetitive description thereof.

The compressive hemostatic belt of the invention is characterised in that the bowl shape of the rigid case 4 is modified to provide a flat pot adapted to hold the balloon 5 and having an inner flange 4a formed thereon at the opening to prevent the balloon from sticking out. With this arrangement, even if the strip 1 is wrapped so that a portion thereof is subjected to a force the balloon 5 is expanded toward the catheter insertion wound A while its sticking out is controlled by the inner flange 4a of the rigid case 4. Thus, since there no possibility of the rigid case 4 floating to deviate from the normal position, the catheter insertion wound A can be reliably compressed through the gauze 10 to completely stop the bleeding from the catheter insertion wound A.

In addition, in the above embodiment, the inner flange 4a of the rigid case 4 is integrally formed, but the invention is not limited thereto and as shown in Fig. 11 the inner flange 4a may be a doughnut-shaped separate part having its compressive area cut out and may be attached to the open end by suitable attaching means such as screwing, fitting, bonding or welding. In the case where the inner flange 4a is removably attached to the rigid case, several inner flanges respectively having their different compressive areas cut out are prepared so that by changing them as the need arises, the compressive area for the patient can be easily adjusted.

In the above embodiment, the rigid case 4 is made of synthetic resin, but it may be formed of leather, thick nonstretch textile fabric or the like.

Another embodiment of the invention will now be described with reference to Figs.12 and 13.

Fig.12 (a) is a plan view of a compressive hemostatic belt. As in the case of the article shown in Fig.3, this compressive hemostatic belt comprises a strip 1 of nonstretch textile fabric and a fabric 2 in the form of a stretch textile fabric sewn to the strip 1 at a predetermined position to form a pocket 3, and a rigid case 4 and a balloon 5 received in said pocket 3. Further, the rigid case 4 is received with its open side directed to the fabric and the balloon 5 is received in the rigid case 4 such that a fluid feed tube 7 having a check valve 6 projects out of the pocket 3.

In the present invention, at least the portion of the strip 1 which covers the region extending from the waist to the hip when it is wrapped around the body has mounted thereon a reinforcing sheet 13 made of cardboard, PET, vinyl, rubber or fabric, and the reinforcing sheet 13 is about 10 to 90 cm long and it is mounted on one side of the pocket 3 at a suitable position. Thereby, it does not develop wrinkles or kinks during wrapping operation, and the strip 1 can be firmly wrapped without having to apply so much force. Further, by suitably changing the material, area and mounting position of the reinforcing sheet 13, the degree of tightness of wrapping can be adjusted, so that the compressive hemostatic belt is suited to various body types of patients, such as slim and fat persons and children. In addition, in the case where the length of the reinforcing sheet is not more than 10 cm, almost no reinforcing effect is developed and if it is not less than 90 cm, it tends to stick out of the strip 1.

The way the compressive hemostatic belt constructed in the manner described above is used will now be described.

First, as in the case of the article shown in Fig.12 (c), the gauze 10 is placed on the catheter insertion wound 11 subsequently to the catheter examination and the pocket 3 is placed thereon with the fabric 2 directed to the wound 11. Subsequently, as shown in Fig.13, the strip 1 is wrapped around the patient's waist and the end is locked as by a fabric adhesive plaster 14, while an a fabric adhesive plaster 15 is applied to a portion of the body and the strip 1 to prevent deviation. Thereafter, a manually operable pump 8 and a pressure gage 9 are connected to the fluid feed tube 7 of the balloon 5, and a liquid (including gels), or air, nitrogen gas, carbon dioxide gas or other gas is injected into the balloon 5 to expand the latter to compress the wound 11.

In addition, said reinforcing sheet 13, as shown in Fig. 12 (b), is mounted on the opposite surface of the strip 1 which contacts the body as by such means as a two-surface tape or sewing. Alternatively, as shown in Fig.12 (c), the portion of the strip 1 associated with the reinforcing sheet 13 may be of double construction with the reinforcing sheet 13 placed in the clearance therebetween. Alternatively, as shown in Fig.12 (d), the reinforcing sheet 13 may be connected to the end of the strip 1 to construct the reinforcing portion entirely of the reinforcing sheet 13.

## Claims

1. A compressive hemostatic belt comprising a strip (1) of non-stretch or low-stretch fabric, a bag (5) adapted to be expanded by being filled with fluid, said bag being attached to said strip at a predetermined position by means of a pocket (3) formed on said strip and capable of holding said bag, characterised in that an opening (12) is formed in the surface of said pocket opposed to a region where bleeding is to be stopped.

2. A compressive hemostatic belt as claimed in Claim 1, characterized in that said bag (5) is made of a material which is soft at ordinary temperature and hardly permeable to fluid.

3. A compressive hemostatic belt as claimed in Claim 1 or 2, characterized in that a rigid member (4) adapted to prevent the bag (5) from expanding toward the side opposite to a region where bleeding is to be stopped is interposed between said bag and said strip (1).

4. A compressive hemostatic belt as claimed in any previous claim characterised in that said bag (5) is a balloon made of rubber.

5. A compressive hemostatic belt as claimed in any preceding Claim, characterized in that the surface of the pocket (3) of said strip (1) is formed of a stretch textile fabric (2).

6. A compressive hemostatic belt as claimed in any previous Claim, characterized in that a fluid feed pump (8) and a pressure gauge (9) are connected to said rubber balloon (5) through a check valve (6).

7. A compressive hemostatic belt as claimed in Claim 6, characterized in that said rubber balloon (5) is expanded by being filled with air.

8. A compressive hemostatic belt as claimed in Claim 7, characterized in that an air feed line (7) to said rubber balloon (5) has connected thereto compressing means (8) and pressure adjusting means (6), said compressing means being composed of an air pump and a pressure gauge.

9. A compressive hemostatic belt as set forth in Claim 5, characterized in that a rigid case (4) in the form of a basin for preventing said balloon (5) from expanding toward the side opposite to a region where bleeding is to be stopped is disposed between said strip (1) and said balloon, said rigid case having an inner flange (4a) formed on its open end to hold said balloon therein.

10. A compressive hemostatic belt as claimed in Claim 11, characterized in that a doughnut-like inner flange (4a), whose compressive area is suited for an affected area where bleeding is to be stopped, is cut out and attached to the open end of said rigid case (4).

11. A compressive hemostatic belt as claimed in Claim 1, characterized in that at least the portion of said strip (1) which covers an affected area extending from the waist to the hip when wrapped around the body is provided with a reinforcing sheet (13).

12. A compressive hemostatic belt as claimed in Claim 13, characterized in that said reinforcing sheet (13) is 10 to 90 cm long and is disposed on one side of the pocket of the strip.

## Patentansprüche

1. Hämostatischer Druckgürtel mit einem Streifen (1) aus einem nichtdehnbaren oder geringfügig dehnbaren Gewebe, einem Beutel (5), der durch Aufnehmen eines Fluids weitbar ist, wobei der Beute! an dem Streifen in einer vorbestimmten Position mittels einer Tasche (3) angebracht ist, die an dem Streifen ausgebildet ist und die in der Lage ist, den Beutel zu halten,
dadurch gekennzeichnet, daß eine Öffnung (12) in der Oberfläche der Tasche ausgebildet ist, die einem Bereich gegenüberliegt, an dem eine Blutung zu stoppen ist.

2. Hämostatischer Druckgürtel nach Anspruch 1,
dadurch gekennzeichnet, daß der Beutel (5) aus einem Material hergestellt ist, welches bei üblicher Temperatur weich und für ein Fluid kaum durchlässig ist.

3. Hämostatischer Druckgürtel nach Anspruch 1 oder 2,
dadurch gekennzeichnet, daß ein steifes Element (4) zwischen dem Beutel und dem Streifen (1) angeordnet ist, wobei das steife Element den Beutel (5) daran hindert, sich in Richtung zu der Seite zu weiten, die einem Bereich gegenüberliegt, an dem eine Blutung zu stoppen ist.

4. Hämostatischer Druckgürtel nach einem der vorstehenden Ansprüche,
dadurch gekennzeichnet, daß der Beutel (5) ein Gummiballon ist.

5. Hämostatischer Druckgürtel nach einem der vorstehenden Ansprüche,
dadurch gekennzeichnet, daß die Oberfläche der Tasche (3) des Streifens (1) aus einem dehnbaren Textilgewebe (2) hergestellt ist.

6. Hämostatischer Druckgürtel nach einem der vorstehenden Ansprüche,
dadurch gekennzeichnet, daß eine Fluidzuführpumpe (8) sowie ein Druckmesser (9) über ein Rückschlagventil (6) mit dem Gummiballon (5) verbunden sind.

7. Hämostatischer Druckgürtel nach Anspruch 6,
dadurch gekennzeichnet, daß der Gummiballon (5) durch Füllen mit Luft geweitet wird.

8. Hämostatischer Druckgürtel nach Anspruch 7,
dadurch gekennzeichnet, daß an eine Luftzuführleitung (7) zu dem Gummiballon (5) Druckmittel (8) und Druckeinstellmittel (6) angeschlossen sind, wobei die Druckmittel durch eine Luftpumpe und einem Druckmesser gebildet sind.

9. Hämostatischer Druckgürtel nach Anspruch 5,
dadurch gekennzeichnet, daß ein steifes Gehäuse (4) in Form eines Beckens zwischen dem Streifen (1) und dem Ballon angeordnet ist, wobei das steife Gehäuse den Ballon (5) daran hindert, sich in Richtung zu der Seite auszudehnen, die einem Bereich gegebenüberliegt, an dem eine Blutung zu stoppen ist, und wobei das steife Gehäuse einen inneren Flansch (4a) aufweist, der an seinem offenen Ende ausgebildet ist, um den Ballon darin zu halten.

10. Hämostatischer Druckgürtel nach Anspruch 11,
dadurch gekennzeichnet, daß ein torusförmiger, innerer Flansch (4a), dessen Druckbereich für einen Wirkungsbereich ausgebildet ist, an dem eine Blutung zu stoppen ist, ausgeschnitten und an dem offenen Ende des steifen Gehäuses (4) angebracht ist.

11. Hämostatischer Druckgürtel nach Anspruch 1,
dadurch gekennzeichnet daß zumindest der Abschnitt des Streifens (1), welcher einen Einwirkungsbereich abgedeckt, der sich von dem Bauch zu der Hüfte erstreckt, wenn der Streifen um den Körper geschlungen ist, mit einer Verstärkungslage (13) versehen ist.

12. Hämostatischer Druckgürtel nach Anspruch 13,
dadurch gekennzeichnet, daß die Verstärkungslage (13) 10 bis 90 cm lang und an einer Seite der Tasche des Streifens angeordnet ist.

## Revendications

1. Ceinture hémostatique de compression comprenant une bande (1) de tissu non extensible ou à faible extensibilité, un sac (5) adapté pour être dilaté en étant rempli de fluide, ledit sac étant fixé à ladite bande, en une position prédéterminée, au moyen d'une poche (3) formée sur ladite bande et pouvant maintenir ledit sac, caractérisée en ce qu'une ouverture (12) est ménagée dans la surface de ladite poche en regard de la zone dans laquelle les saignements doivent être arrêtés.

2. Ceinture hémostatique de compression selon la revendication 1, caractérisée en ce que ledit sac (5) est constitué d'un matériau qui est souple à température ambiante et étanche aux fluides.

3. Ceinture hémostatique de compression selon la revendication 1 ou la revendication 2, caractérisée en ce qu'un élément rigide (4) adapté pour empêcher le sac (5) de se dilater en direction du côté, situé en regard de la zone dans laquelle les saignements doivent être arrêtés, est interposé entre ledit sac et ladite bande (1).

4. Ceinture hémostatique de compression selon l'une quelconque des revendications précédentes, caractérisée en ce que ledit sac (5) est un ballonnet de caoutchouc.

5. Ceinture hémostatique de compression selon l'une quelconque des revendications précédentes, caractérisée en ce que la surface de la poche (3) de ladite bande (1) est constituée d'un tissu extensible (2).

6. Ceinture hémostatique de compression selon l'une quelconque des revendications précédentes, caractérisée en ce qu'une pompe d'alimentation en fluide (8) et un manomètre (9) sont connectés audit ballonnet de caoutchouc (5) par l'intermédiaire d'un clapet anti-retour (6).

7. Ceinture hémostatique de compression selon la revendication 6, caractérisée en ce que ledit ballonnet de caoutchouc (5) est dilaté en étant rempli d'air.

8. Ceinture hémostatique de compression selon la revendication 7, caractérisée en ce qu'une conduite d'alimentation en air (7), reliée au ballonnet de caoutchouc (5), est également raccordée à des moyens de compression (8) et des moyens de réglage de la pression (6), lesdits moyens de compression étant composés d'une pompe à air et d'un manomètre.

9. Ceinture hémostatique de compression selon la revendication 5, caractérisée en ce qu'un boîtier rigide (4) en forme de cuvette, prévu pour empêcher ledit ballonnet (5) de se dilater en direction du côté situé en regard de la zone dans laquelle les saignements doivent être arrêtés, est placé entre ladite bande (1) et ledit ballonnet, ledit boîtier rigide présentant un rebord intérieur (4a) formé sur son extrémité ouverte pour maintenir ledit ballonnet en position.

10. Ceinture hémostatique de compression selon la revendication 11, caractérisée en ce qu'un rebord intérieur (4a) en forme de beignet, dont la zone de compression est appropriée pour une zone affectée dans laquelle les saignements doivent être arrêtés, est découpé et fixé à l'extrémité ouverte dudit boîtier rigide (4).

11. Ceinture hémostatique de compression selon la revendication 1, caractérisée en ce qu'au moins la partie de ladite bande (1) qui recouvre une zone affectée s'étendant depuis la taille jusqu'à la hanche, une fois enroulée autour du corps, est pourvue d'une feuille de renforcement (13).

12. Ceinture hémostatique de compression selon la revendication 13, caractérisée en ce que la dite feuille de renforcement (13) présente une longueur de 10 à 90 cm et est placée sur un côté de la poche de la bande.
